# EUROPÄISCHE PATENTSCHRIFT

(11) **EP 3 141 222 B1**
(45) Veröffentlichungstag und Bekanntmachung des Hinweises auf die Patenterteilung: **06.02.2019**
(21) Anmeldenummer: 15184164.0
(22) Anmeldetag: 08.09.2015
(51) Int. Cl.: A61F 2/44

(54) **IMPLANTAT**
IMPLANT
IMPLANT

(43) Veröffentlichungstag der Anmeldung: 15.03.2017
(73) Patentinhaber: Ulrich GmbH & Co. KG, 89081 Ulm (DE)
(72) Erfinder: SCHILLING, Julia, 89231 Neu-Ulm (DE); SCHRÖTER, Martin, 89264 Weissenhorn (DE); WINKLER, Tobias, 89160 Dornstadt (DE); CHRISTENHUSZ, Harry, 48455 Bad Bentheim (DE); TER BRAAK, Hubertus Paul Maria, 7482MA Haaksbergen (NL)
(74) Vertreter: Hentrich Patentanwälte PartG mbB

(56) Entgegenhaltungen:
- US-A1- 2003 045 877
- US-A1- 2010 324 687
- US-A1- 2014 277 510

## Beschreibung

Die Erfindung betrifft ein Implantat zum Einbringen zwischen Wirbelkörper der Wirbelsäule gemäß dem Oberbegriff des Patentanspruchs 1.

Derartige Implantate sind beispielsweise aus der DE 44 17 629 A1 bekannt, die ein Wirbelsäulenimplantat mit einem Hauptkörper beschreibt, an dessen beiden Enden eine Ansatzplatte angeordnet ist, die nach Einstellung der Winkellage bezüglich des Hauptkörpers mit diesem starr verbindbar ist. Nachteilig ist hierbei, dass nach der Einstellung des Winkels die Fixierung desselben entweder durch das Einfüllen von Knochenmaterial oder durch das Einbringen eines aushärtebaren Materials erfolgt. Dies stellt ein aufwendiges Verfahren zur nachträglichen Fixierung des eingestellten Winkels dar und ist darüber hinaus auch irreversibel. Eine nachträgliche Änderung des Winkels kann nach der Fixierung nicht korrigiert werden.

Weiterhin ist die US 2010/0324687 bekannt, die ein Wirbelimplantat mit winkelverstellbarer Ansatzplatte zeigt, wobei zwischen Ansatzplatte und Hauptkörper ein Klemmelement angeordnet, welches mit Hilfe einer zwischen Klemmelement und Hauptkörper angeordneten Feder an die Ansatzplatte gedrückt wird und diese gegenüber dem Hauptkörper in der gewünschten Winkeleinstellung blockiert. Das Klemmelement weist dabei zwei geneigte Oberflächen mit einer diese trennenden scharfen Kante auf, die in Eingriff ist mit einer an der dem Hauptkörper zugewandten Seite der Ansatzplatte ausgebildete Verzahnung. Die Winkeleinstellung der Ansatzplatte erfolgt, indem die Ansatzplatte über die Verzahnung entlang der geneigten Oberfläche des Klemmelements kämmt.

Darüber hinaus ist die US 2014/0277510 bekannt, die ebenfalls einen Wirbelkörper mit winkelverstellbarer Ansatzplatte zeigt. Die Winkelverstellung erfolgt dabei, indem die Ansatzplatte mit dem Hauptkörper über ein Lager schwenkbar verbunden ist. Die Fixierung der Winkellage erfolgt durch das Einschieben eines Keils zwischen Hauptkörper und Ansatzplatte.

Aufgabe der vorliegenden Erfindung ist es daher, ein Implantat bereitzustellen, welches besonders einfach betätigt werden kann.

Diese Aufgabe wird nach der Erfindung bei einem Implantat gemäß Patentanspruch 1 gelöst. Durch
die Wechselwirkung zwischen dem Klemmelement und der Klemmfläche kann durch eine Klemmverbindung der Winkel der Ansatzplatte gegenüber der Längsachse auf eine sehr einfache Art und Weise fixiert werden. Das zeitaufwendige Fixieren des Winkels der Ansatzplatte durch Schrauben oder durch das Einbringen von Fixiermaterial entfällt somit. Die schwenkbare Lagerung der Ansatzplatte gegenüber dem Hauptkörper ermöglicht darüber hinaus eine stufenlose und damit sehr feine und genaue Justage des optimalen Winkels. Durch die Verstellbarkeit des Klemmelements zwischen einer Schwenkkonfiguration und einer Klemmkonfiguration ist es möglich den Winkel reversibel zu fixieren, das heißt einen bereits vorher eingestellten und fixierten Winkel zwischen Ansatzplatte und Längsachse später korrigieren bzw. optimieren zu können. Dies ermöglicht dem Operateur auf eine schnelle Art und Weise den optimalen Winkel zu finden und einzustellen, so dass die Anlagefläche optimal am Wirbelkörper anliegt. Dadurch wird auch das Einbrechen des Wirbelkörperersatzes in den angrenzenden Wirbelkörper durch eine optimale Lastverteilung verhindert. Alternativ oder zusätzlich ist es günstig, wenn der Winkel irreversibel fixierbar ist, das Klemmelement also, wenn es in die finale Klemmkonfiguration verstellt wurde, nicht mehr in die Schwenkkonfiguration zurückstellbar ist. Im Rahmen der Erfindung ist es weiterhin vorteilhaft, dass jedem Ende des Hauptkörpers eine Ansatzplatte mit einer Anlagefläche zur Anlage an den angrenzenden Wirbelkörper zugeordnet ist, die jeweils um eine im Wesentlichen senkrecht zur Längsachse angeordnete Schwenkachse schwenkbar am Hauptkörper gelagert und unter einem Winkel gegenüber diesem oder gegenüber der Längsachse fixierbar sind, dass dem Hauptkörper für jede der Ansatzplatten mindestens ein Klemmelement zugeordnet ist, dass die Ansatzplatten an einer dem Hauptkörper zugewandten Seite jeweils mindestens eine Klemmfläche aufweisen und dass die Klemmelemente verstellbar sind zwischen einer Schwenkkonfiguration, in der die Ansatzplatten schwenkbar sind und einer Klemmkonfiguration, in der durch die Wechselwirkung der Klemmelemente mit den Klemmflächen der Winkel und damit die Ansatzplatten fixiert sind. Dies ermöglicht, dass das Implantat noch genauer an die Ausbildung und die Orientierung der angrenzenden Wirbelkörper angepasst werden kann.

In einer bevorzugten Ausführungsform weist die Ansatzplatte mehrere Klemmflächen an einer dem Hauptkörper zugewandten Seite auf. Dabei können die Klemmflächen entlang der Längsachse der Ansatzplatte oder aber in einer bevorzugten Ausführungsform annähernd senkrecht zur Längsachse ausgebildet sein. Der Hauptkörper weist in einer vorteilhaften Ausführungsform an der der Ansatzplatte zugewandten Seite einen Rand auf. Dieser dient unter anderem der Beschränkung des einstellbaren Winkels und dient als Anschlag für das Klemmelement. Weiterhin ist der Hauptkörper vorzugsweise als ein Hohlkörper gebildet und weist umfangsseitig an der Mantelfläche des Hauptkörpers einen oder mehrere Ausnehmungen auf.

In einer besonders einfachen Ausführungsform ist das Klemmelement als ein Klemmschwenker gebildet und jeder Ansatzplatte sind zwei der Klemmschwenker zugeordnet, die schwenkbar am Hauptkörper gelagert sind. Durch Schwenken der Klemmschwenker um eine Klemmachse kommt es im Rahmen der Klemmkonfiguration zu einer Klemmverbindung zwischen den Klemmschwenkern und den Klemmflächen der Ansatzplatte.

In einer alternativen Ausführungsform kann das Klemmelement als eine Hülse gebildet sein, die am Hauptkörper gelagert ist und in und entgegen der Richtung der Ansatzplatte verschiebbar ist. Durch das Verschieben der Hülse entlang der Längsachse des Hauptkörpers kann wiederum eine Klemmverbindung zwischen der Klemmfläche der Ansatzplatte und der Hülse gebildet werden.

In einer weiteren alternativen Ausführungsform kann das Klemmelement als ein Schiebeelement gebildet sein, welches in und entgegen der Richtung der Ansatzplatte am Hauptkörper entlang der Längsachse verschoben werden kann und so, vorzugsweise reversibel eine Klemmverbindung mit der Klemmfläche eingehen kann.

Im Rahmen der Erfindung ist es ferner vorgesehen, dass das Klemmelement um eine im Wesentlichen senkrecht zur Längsachse angeordnete Achse schwenkbar am Hauptkörper gelagert ist. Durch die schwenkbare Lagerung des Klemmelements am Hauptkörper kann eine besonders schnelle Einstellung bzw. Fixierung der Klemmkonfiguration erfolgen. Dabei ist es in einer Ausführungsform vorgesehen, dass das Klemmelement und die Ansatzplatte um dieselbe Schwenkachse schwenkbar gelagert sind. Alternativ und in einer bevorzugten Ausführungsform ist das Klemmelement um eine senkrecht zur Längsachse orientierte Achse schwenkbar, die als eine Klemmachse gebildet ist, wobei vorzugsweise die Schwenkachse und die Klemmachse im Wesentlichen parallel zueinander orientiert sind. Weiterhin ist es im Rahmen der Erfindung vorgesehen, dass das Klemmelement als ein Klemmbügel gebildet ist, der den Hauptkörper umfangsseitig zumindest teilweise umgreift. Die Ausbildung des Klemmelements als ein Klemmbügel bildet ein besonders einfach handhabbares Klemmelement, das auf einfache Weise zwischen der Klemmkonfiguration und der Schwenkkonfiguration verstellbar ist. In einer Ausführungsform, in der die Klemmkonfiguration irreversibel vorgesehen ist, ist am Klemmbügel vorzugsweise eine Sollbruchstelle im Material beispielsweise in Form einer Einkerbung gebildet, so dass der Klemmbügel nach der endgültigen Fixierung des Winkels abgebrochen werden kann.

Insbesondere ist es vorteilhaft, wenn am Außenumfang des Hauptkörpers ein Fortsatz ausgebildet oder angeordnet ist, welcher insbesondere eine zumindest teilweise planare Sicherungsfläche umfasst. Dieser Fortsatz dient der zusätzlichen Sicherung der Klemmelements bzw. des Klemmbügels in der Klemmkonfiguration. Dabei wird der Klemmbügel bei der Verstellung in die Klemmkonfiguration über die Sicherungsfläche geschoben, wodurch die Sicherungsfläche und der Klemmbügel vorzugsweise einen Kraftschluss bilden. Die Sicherungsfläche kann dabei auch als eine Sicherungslinie gebildet sein.

In diesem Zusammenhang ist es vorgesehen, dass der Fortsatz unter einem Neigungswinkel geneigt oder im Wesentlichen parallel zur Längsachse ausgerichtet ist. Im Falle eines unter einem Neigungswinkel geneigten Fortsatzes ist es vorstellbar, dass der Fortsatz ein Plateau aufweist auf dem das Klemmelement in der Klemmkonfiguration anliegt.

Ganz besonders vorteilhaft ist es, wenn dem Klemmelement mindestens ein Kopplungsglied an der der Klemmfläche zugewandten Seite zugeordnet ist zur zusätzlichen Sicherung der Klemmkonfiguration. Das Kopplungsglied kann dabei als ein Rastglied gebildet sein mit einem an der Klemmfläche ausgebildeten Rastsitz. Besonders bevorzugt ist es allerdings, wenn das Kopplungsglied als ein einzelner oder als mehrere Dorne gebildet ist. Dabei ist es insbesondere günstig, wenn die Dorne in die Klemmfläche einschneiden können zur zusätzlichen Sicherung der Klemmkonfiguration.

Weiterhin ist es gemäß einer weiteren Alternative vorgesehen, dass die Ansatzplatte und der Hauptkörper durch eine Schwenkeinheit miteinander verbunden sind, die eine dem Hauptkörper zugewandte Ausbuchtung mit einer Verbindungsgliedaufnahme, die eine am Hauptkörper ausgebildete Ausbuchtungsaufnahme mit einer Verbindungsgliedöffnung und die ein in die Verbindungsgliedaufnahme sowie in die Verbindungsgliedöffnung eingesetztes Verbindungsglied umfasst. Die Schwenkeinheit stellt dabei eine konstruktiv besonders einfache Lösung dar, die Ansatzplatte schwenkbar am Hauptkörper zu lagern. Dabei wird die Schwenkbewegung der Ansatzplatte durch die Schwenkbewegung der Ausbuchtung in der Ausbuchtungsaufnahme geführt. Dadurch kann der Winkel besonders fein bzw. genau eingestellt werden. Insbesondere ist es vorteilhaft, wenn das Verbindungsglied als Bolzen oder Stift gebildet ist. Weiterhin ist es vorgesehen, dass die Verbindungsgliedöffnung als ein Langloch gebildet ist. In einer anderen Ausgestaltung der Erfindung ermöglicht das Langloch eine begrenzte laterale Verschiebbarkeit des Verbindungsglieds im Langloch und damit der Ansatzplatte gegenüber dem Hauptkörper. Dadurch wird der Schwenkwinkel der Ansatzplatte um die Schwenkachse vergrößert und das Implantat kann noch optimaler an die Ausbildung und Orientierung der angrenzenden Wirbelkörper angepasst werden. Weiterhin ist es bevorzugt, wenn jede Ansatzplatte über zwei Schwenkeinheiten mit dem Hauptkörper verbunden ist und wenn die Schwenkeinheiten gleichmäßig über den Umfang der Ansatzplatte bzw. des Hauptkörpers verteilt angeordnet sind. Alternativ ist es vorgesehen, dass die als Langloch gebildete Verbindungsgliedöffnung an der Ansatzplatte ausgebildet ist, dass am Hauptkörper eine Verbindungsgliedaufnahme angeordnet ist und dass ein als Stift gebildetes Verbindungsglied in der Verbindungsgliedaufnahme und der Verbindungsgliedöffnung aufgenommen ist.

In diesem Zusammenhang ist es bevorzugt, wenn die Ausbuchtung zumindest teilweise konvex gebildet ist und wenn die Ausbuchtungsaufnahme vorzugsweise zumindest teilweise konkav gebildet ist. Hierdurch kann die Schwenkbewegung besonders gut geführt werden, wobei es insbesondere vorgesehen ist, dass die Ausbuchtung konvex und die Ausbuchtungsaufnahme dazu passend konkav gebildet ist. Mit anderen Worten bilden die Ausbuchtung und die Ausbuchtungsaufnahme ein Gelenk aus. Dieses ermöglicht eine besonders genaue Einstellung des Winkels und damit auch eine besonders einfache Fixierung des Winkels. Im Rahmen dessen ist es günstig, wenn lediglich Teile der konvexen Ausbuchtung in der konkaven Ausbuchtungsaufnahme aufgenommen werden. In einer alternativen Ausführungsform kann die Ausbuchtungsaufnahme auch eben gebildet sein.

In einer alternativen Ausführungsform ist es vorgesehen, dass die Ansatzplatte und der Hauptkörper durch eine Schwenkeinheit miteinander verbunden sind, die ein Schwenkglied und eine am Hauptkörper ausgebildete Schwenkgliedaufnahme umfasst. Das Schwenkglied kann dabei beispielsweise als Stift oder Bolzen gebildet sein, das in die Schwenkgliedaufnahme eingreift und darin schwenkbar gelagert ist.

Im Rahmen der Erfindung ist es weiterhin vorgesehen, dass das Klemmelement und der Hauptkörper durch ein Schwenkmittel miteinander verbunden sind, das eine am Klemmelement ausgebildete Klemmelementverbindungsgliedaufnahme, das eine am Hauptkörper ausgebildete Klemmelementverbindungsgliedöffnung und das ein in die Klemmelementverbindungsgliedaufnahme sowie in die Klemmelementverbindungsgliedöffnung eingesetztes Klemmelementverbindungsglied umfasst. Dies ermöglicht, dass das Klemmelement schwenkbar am Hauptkörper gelagert ist. Dabei ist es besonders vorteilhaft, wenn das Klemmbügelverbindungsglied als Bolzen oder Stift gebildet ist. Weiterhin ist es vorgesehen, dass jedem Klemmelement ein Schwenkmittel zugeordnet ist und dass die Schwenkmittel gleichmäßig über den Umfang des Hauptkörpers verteilt angeordnet sind. Auch ist eine Art eines Schwenkgelenks gebildet. Wenn das Klemmelement als Klemmbügel gebildet ist, dann ist es bevorzugt, wenn der Klemmbügel über genau zwei Schwenkmittel mit dem Hauptkörper verbunden ist. Weiterhin ist es bevorzugt, dass das Klemmelement im Bereich des Schwenkmittels mit einem größeren Durchmesser gebildet ist. Dies ermöglicht eine höhere Belastbarkeit des Klemmelements und verhindert das Abbrechen des Klemmelements bei dessen Verstellung in die Klemmkonfiguration.

In diesem Zusammenhang hat es sich als günstig erwiesen, wenn der Hauptkörper an dem der Schwenkeinheit zugewandten Ende eine Verstärkung aufweist, in der die Ausbuchtungsaufnahme mit der Verbindungsgliedöffnung und die Klemmbügelverbindungsgliedöffnung ausgebildet sind. Dabei erstreckt sich die Verstärkung zumindest teilweise umfangsseitig am Hauptkörper. Die Verstärkung verbessert die Belastbarkeit des Implantats und verhindert, dass die Ansatzplatte beim Implantieren und Fixieren vom Hauptkörper abbricht.

Ganz besonders vorteilhaft ist es, wenn der Hauptkörper eine Klemmbügelaufnahme aufweist zur Aufnahme eines am Klemmbügel ausgebildeten Klemmbügelfortsatzes. Dieser dient der Sicherung des Klemmbügels am Hauptkörper in der Schwenkkonfiguration. Es hat sich weiterhin als sinnvoll erwiesen, wenn am Hauptkörper ein federndes Element angeordnet ist zur Sicherung des Klemmelements in der Klemmkonfiguration. Vorzugsweise ist das federnde Element als ein Schnapper gebildet mit einem radial nach außen abragenden Ende. Weiterhin ist es bevorzugt, wenn das federnde Element in der Schwenkkonfiguration vorzugsweise radial vorgespannt ist.

Im Rahmen der Erfindung ist es weiterhin bevorzugt, wenn das federnde Element in der Schwenkkonfiguration axial auf den Klemmbügelfortsatz wirkt und dass der Klemmbügelfortsatz in der Klemmkonfiguration radial mit der Klemmbügelaufnahme zusammenwirkt. Dabei ist der Klemmbügelfortsatz entlang der Längsachse des Hauptkörpers auf dem federnden Element aufliegend vorgesehen. Dies sichert den Klemmbügel in der Schwenkkonfiguration. Das radiale Zusammenwirken des Klemmbügelfortsatzes und der Klemmbügelaufnahme in der Klemmkonfiguration dient der zusätzlichen Sicherung der Klemmkonfiguration bzw. der Klemmverbindung.

Zusammenfassend liegt der Vorteil der vorliegenden Erfindung darin, dass das Implantat einfach gehandhabt werden kann und somit auf eine vergleichsweise einfache Art und Weise intervertebral implantiert und optimal gegenüber den angrenzenden Wirbelkörpern ausgerichtet werden kann. Dadurch ist das Implantat entlang der Wirbelsäule variabel einsetzbar. Durch die schwenkbar am Hauptkörper gelagerte Ansatzplatte kann auf einfache Weise ein Winkel der Ansatzplatte gegenüber dem Hauptkörper festgelegt werden, so dass die Ansatzplatte optimal gegenüber den angrenzenden Wirbelkörpern ausgerichtet ist. Durch die Wechselwirkung zwischen dem Klemmelement und der an der Ansatzplatte angeordneten Klemmflächen wird ein Kraftschluss zwischen Klemmelement und Klemmfläche gebildet bzw. eine Klemmverbindung ausgebildet. Diese Klemmverbindung ermöglicht es, den zuvor gewählten Winkel zeitsparend und sicher festzulegen bzw. zu fixieren. Die Verstellung des Klemmelementes zwischen der Klemmkonfiguration und der Schwenkkonfiguration, sowie die Einstellung des Winkels in der Schwenkkonfiguration benötigt keine bzw. nur sehr wenige zusätzliche Instrumente. Das Implantat ist folglich besonders leicht und auf einfache Weise an die Bedingungen an der Wirbelsäule des menschlichen Körpers anpassbar.

Im Folgenden wird die Erfindung an einem in der Zeichnung dargestellten Ausführungsbeispiel näher erläutert; es zeigen:
- Fig. 1: eine perspektivische Darstellung des erfindungsgemäßen Implantats in der Schwenkkonfiguration,
- Fig. 2: eine Vorderansicht des erfindungsgemäßen Implantats in der Schwenkkonfiguration,
- Fig. 3: eine Seitenansicht des erfindungsgemäßen Implantats in der Schwenkkonfiguration,
- Fig. 4: eine Explosionsdarstellung des erfindungsgemäßen Implantats,
- Fig. 5: eine perspektivische Darstellung des erfindungsgemäßen Implantats in der Klemmkonfiguration und
- Fig. 6: eine Seitenansicht des erfindungsgemäßen Implantats in der Klemmkonfiguration.

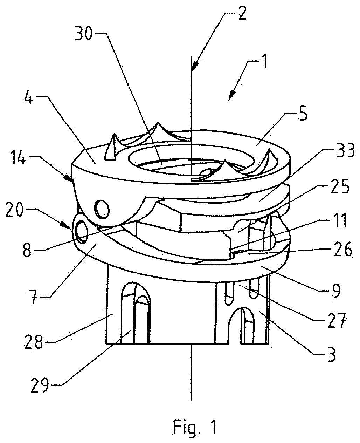

Figur 1 zeigt eine perspektivische Darstellung des erfindungsgemäßen Implantats 1, mit einem entlang einer Längsachse 2 ausgebildeten Hauptkörper 3, an dessen einem Ende eine Ansatzplatte 4 mit einer Anlagefläche 5 zur Anlage an einen angrenzenden Wirbelkörper angeordnet ist. Der Hauptkörper 3 ist als ein Hohlkörper gebildet mit mehreren radial in Umfangsrichtung an der Mantelfläche 28 ausgebildeten Ausnehmungen 29 zur Gewichtsreduzierung bzw. zur Bereitstellung von Durchtritten für Knochenmaterial.

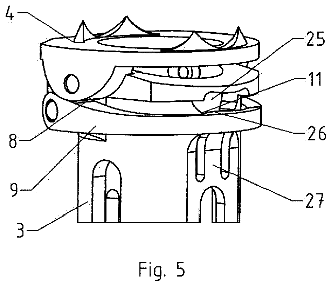

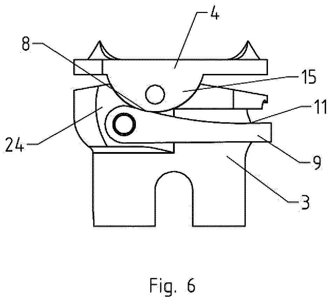

In den Figuren 1 bis 6 ist der Hauptkörper 3 entlang seiner Längsachse 2 ausschnittsweise dargestellt. Die Ansatzplatte 4 weist an ihrer Anlagefläche 5 scharfe Zähne 34 als Dislokationsschutz gegenüber dem Wirbelkörper auf. Auch die Ansatzplatte 4 weist mindestens eine, vorliegend ebenfalls in Längsrichtung ausgebildete Ansatzplattenausnehmung 30 auf, die vorzugsweise dem Einwachsen von Knochenmaterial oder dem Einbringen von Knochenzement dient.

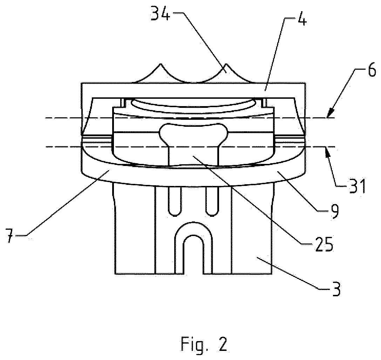

Die Ansatzplatte 4 ist dabei um eine im Wesentlichen senkrecht zur Längsachse 2 angeordnete Schwenkachse 6 schwenkbar am Hauptkörper 3 gelagert und unter einem Winkel gegenüber diesem oder gegenüber der Längsachse 2 fixierbar (vgl. Fig. 2).

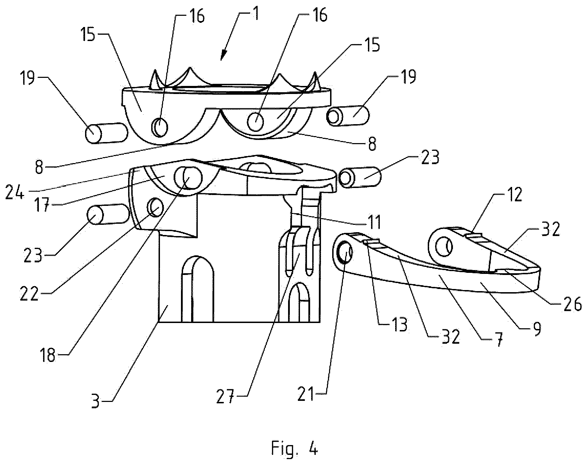

Die schwenkbare Lagerung der Ansatzplatte 4 am Hauptkörper 3 erfolgt dabei durch eine Schwenkeinheit 14, die eine dem Hauptkörper 3 zugewandte und an der Ansatzplatte 4 ausgeprägte Ausbuchtung 15 mit einer Verbindungsgliedaufnahme 16, eine im Hauptkörper 3 ausgebildete Ausbuchtungsaufnahme 17 mit einer Verbindungsgliedöffnung 18 und ein in die Verbindungsgliedaufnahme 16 sowie in die Verbindungsgliedöffnung 18 einsetzbares Verbindungsglied 19 umfasst. Die einzelnen Bestandteile der Schwenkeinheit 14 sind genauer in Figur 4 zu erkennen. Die Ausbuchtung 15 ist vorliegend im Wesentlichen konvex gebildet, während die Ausbuchtungsaufnahme 17 im Wesentlichen dazu passend konkav gebildet ist. Die Ausbuchtungsaufnahme 17 ist in Form einer am Hauptkörper 3 ausgebildeten Schulter geformt, die die Ausbuchtung 15 aufnimmt bzw. abstützt.

Die Verbindungsgliedöffnung 18 ist vorliegend als ein, insbesondere radial bezüglich der Längsachse 2 ausgeprägtes, Langloch gebildet. Das Verbindungsglied 19 ist hingegen als ein im Querschnitt runder Stift gebildet, wobei ebenfalls ein im Querschnitt unrunder Bolzen als Verbindungsglied 19 infrage kommt. Der Stift bzw. das Verbindungsglied 19 dient der Fixierung der Ansatzplatte 4 am Hauptkörper 3. Mit anderen Worten ist also der Stift primär derart ausgelegt, die Ansatzplatte 4 mit dem Hauptkörper 3 zu verbinden und ein Abheben der Ansatzplatte 4 vom Hauptkörper 3 zu verhindern. Sekundär wird der maximale Schwenkwinkel bei der vorliegenden Erfindung begrenzt.

Der Ansatzplatte 4 sind vorliegend mehrere, insbesondere genau zwei gleichmäßig über den Umfang verteilte Schwenkeinheiten 14 zugeordnet. Die Schwenkeinheiten 14 sind dabei um eine gemeinsame Schwenkachse 6 verschwenkbar. Darüber hinaus weist die Ansatzplatte 4 auf ihrer dem Hauptkörper 3 zugewandten Seite mehrere, vorliegend genau zwei, insbesondere gleichmäßig über den Umfang verteilte Klemmflächen 8 auf. Diese sind an der dem Hauptkörper 3 zugewandten Seite der beiden Ausbuchtungen 15 ausgebildet.

Um nun den Winkel zwischen der Ansatzplatte 4 bezüglich der Längsachse 2 bzw. bezüglich des Hauptkörpers 3 fixieren zu können, ist ein Klemmelement 7 vorgesehen, was um eine im Wesentlichen senkrecht zur Längsachse 2 orientierte Klemmachse 31 schwenkbar am Hauptkörper 3 gelagert ist. Das Klemmelement 7 ist vorliegend als ein Klemmbügel 9 gebildet, der den Hauptkörper 3 umfangsseitig zumindest teilweise umgreift.

Der Klemmbügel 9 zeigt mehrere, vorliegend genau zwei Klemmbügelschenkel 32 auf, und ist zwischen zwei Konfigurationen verstellbar am Hauptkörper 3 gelagert. Der Klemmbügel 9 ist verstellbar zwischen einer Schwenkkonfiguration, in der die Ansatzplatte 4 schwenkbar ist und eine Klemmkonfiguration, in der durch eine Wechselwirkung des Klemmelements 7 mit den Klemmflächen 8, der Winkel und damit die Ansatzplatte 4 fixierbar ist.

Das Klemmelement 7 bzw. der Klemmbügel 9 ist dabei durch ein Schwenkmittel 20 am Hauptkörper 3 schwenkbar gelagert, wobei das Schwenkmittel 20 eine im Klemmelement 7 ausgebildete Klemmelementverbindungsgliedaufnahme 21, eine am Hauptkörper 3 ausgebildete Klemmelementverbindungsgliedöffnung 22 und ein in die Klemmelementverbindungsgliedaufnahme 21 sowie in die Klemmelementverbindungsgliedöffnung 22 eingesetztes Klemmelementverbindungsglied 23 umfasst.

Der Klemmbügel 9 weist mehrere, vorliegend genau zwei der Schwenkmittel 20 auf, wobei der Klemmbügel 9 zur Erhöhung der Stabilität im Bereich des Schwenkmittels 20 eine Verstärkung aufweist. Darüber hinaus weist der Klemmbügel 9 an jedem der Klemmbügelschenkel 32 ein Kopplungsglied 12 auf, welches als ein Dorn 13 gebildet ist und welcher in einer Ausführungsform der Erfindung in die Klemmfläche 8 an der Ausbuchtung 15 einschneiden kann. Mit anderen Worten bildet der Klemmbügel 9 in der Klemmkonfiguration eine feste Verbindung mit der Klemmfläche 8 der Ansatzplatte 3 aus. Diese feste Verbindung kann ein Kraftschluss, ein Formschluss oder auch ein Stoffschluss (z.B: Adhäsionsverschluss) sein. Weiterhin weist der Klemmbügel 9 einen Klemmbügelfortsatz 26 auf, der in einer am Hauptkörper 3 ausgebildeten Klemmbügelaufnahme 25 aufnehmbar ist. In der Klemmbügelaufnahme 25 ist wiederum zusätzlich ein federndes Element 27 in Form eines Schnappers ausgebildet, der ein radial nach außen abragendes Ende aufweist und der in der Schwenkkonfiguration vorzugsweise einer radialen Vorspannung unterliegt. In der Schwenkkonfiguration liegt der Klemmbügelfortsatz 26 auf dem radial nach außen abragenden Ende des federnden Elementes 27 auf, so dass das federnde Element 27 den Klemmbügel 9 in der Klemmkonfiguration sichert. Eine alternative Ausführungsform sieht vor, dass das Element 27 ohne eine Federvorspannung ausgeführt ist, welches dann manuell radial nach außen bzw. nach innen, d.h. zwischen der sichernden und entsichernden Position verstellbar ist, um wiederum den Klemmbügel in der Klemmkonfiguration zu sichern bzw. zu entsichern.

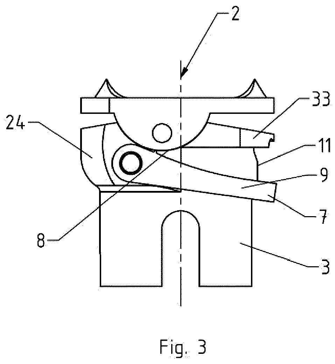

Weiterhin ist am Außenumfang des Hauptkörpers 3 ein Fortsatz 10 ausgebildet, (vgl. Fig. 3) der eine planare Sicherungsfläche 11 umfasst. Die planare Sicherungsfläche 11 ist dabei parallel zur Längsachse 2 ausgerichtet und dient der zusätzlichen Sicherung des Klemmbügels 9 in der Klemmkonfiguration, indem der Klemmbügel 9 über den Fortsatz 10 geschoben wird und so eine feste Verbindung, vorzugsweise einen Kraftschluss mit dem Fortsatz 10 bildet.

Bei der vorliegenden Ausführungsform weist der Hauptkörper 3 mehrere, insbesondere zwei planare Sicherungsflächen 11 auf, die umfangsseitig im Anschluss an die Klemmbügelaufnahme 25 ausgebildet sind. Der Hauptkörper 3 weist weiterhin umfangsseitig, hauptsächlich im Bereich der Schwenkeinheit 14, eine Verstärkung 24 auf, in der die Ausbuchtungsaufnahme 17 mit der Verbindungsgliedöffnung 18 und die Klemmelementverbindungsgliedöffnung 22 ausgebildet sind. Diese Verstärkung 24 ermöglicht eine robuste Bauweise und verhindert das Herausbrechen der Ansatzplatte 4 aus dem Hauptkörper 3. Durch die selektive Ausbildung der Verstärkung 24 lediglich an der Schwenkeinheit 14 wird wiederum Material eingespart. Weiterhin ist die Verstärkung 24 umfangsseitig auch in Form eines Randes 33 ausgebildet, der zusätzlich die Winkelstellung der Ansatzplatte 4 gegenüber dem Hauptkörper 3 begrenzt.

Im Folgenden werden das Einsetzen und das Anpassen des erfindungsgemäßen Implantats 1 zwischen zwei Wirbelkörper beschrieben: Nach dem Einsetzen des Implantats 1 zwischen zwei Wirbelkörper der Wirbelsäule mit Hilfe eines nicht näher dargestellten Instrumentes, wird die am Hauptkörper 3 schwenkbar gelagerte Ansatzplatte 4, welche über die Anlagefläche 5 mit dem Wirbelkörper in Kontakt steht, um einen Winkel verstellt, so dass die Anlagefläche 5 optimal gegenüber dem Wirbelkörper ausgerichtet ist. Dadurch kann eine stabile und komfortable intervertebrale Verbindung hergestellt werden. Die Verstellung der Ansatzplatte 4 benötigt dabei kein zusätzliches Anziehen von Schrauben oder anderen Fixiermitteln. Die Ansatzplatte 3 kann um die Schwenkachse 6 mittels der Schwenkeinheit 14 um einen Winkel verkippt werden. Ist der optimale Winkel gefunden, so wird der Klemmbügel 9 in Richtung der Ansatzplatte 4 verschwenkt, wodurch die an dem Klemmbügel 9 ausgebildeten Dorne 13 in die Klemmfläche 8 an der Ausbuchtung 15 einschneiden und somit einen Formschluss mit der Klemmfläche 8 bilden. Zusätzlich wirkt aber auch die dem Hauptkörper 3 zugewandte Klemmfläche 8 mit dem am Hauptkörper 3 verschwenkbar gelagerten Klemmbügel 9 zusammen und bildet eine Klemmverbindung, insbesondere einen Kraftschluss aus. Das Klemmelement 9 besitzt somit also eine der Ansatzplatte 4 zugewandte Gegenklemmfläche, die die Klemmfläche 8 der Ansatzplatte 4 in der Klemmkonfiguration beaufschlagt. Der Klemmbügel 9 wird durch das federnde Element 27 und/oder durch einen Kraftschluss (oder auch durch einen Reibschluss) zwischen den an der Außenseite des Hauptkörpers 3 ausgebildeten planaren Sicherungsflächen 11 in seiner Klemmkonfiguration gesichert.

Die Sicherung der Klemmkonfiguration erfolgt gemäß einer bevorzugten Variante der Erfindung folglich durch bis zu vier Mechanismen:
1. Die vorzugsweise axiale Sicherung des Klemmbügels 9 in der Klemmkonfiguration durch das vorzugsweise radial verstellbare federnde Element 27,
2. alternativ oder ergänzend durch einen Kraftschluss zwischen dem Klemmbügel 9 und der Klemmfläche 8,
3. alternativ oder ergänzend durch einen Formschluss zwischen den Dornen 13 und der Klemmflächen 8 und
4. alternativ oder ergänzend durch einen Kraftschluss zwischen dem Klemmbügel 9 und den Sicherungsflächen 11.

Im Falle einer reversiblen Klemmkonfiguration kann der eingestellte und fixierte Winkel im Nachgang noch einmal korrigiert bzw. optimiert werden, indem der Klemmbügel 9 wieder in Richtung des Hauptkörper 3 verstellt bzw. verschwenkt wird und sich somit die Klemmverbindung zwischen Klemmbügel 9 und Klemmfläche 8 löst. Außerdem hat sich der Formschluss zwischen den Dornen 13 und der Klemmflächen 8, sowie der Kraftschluss zwischen dem Klemmbügel 9 und den Sicherungsflächen 11 zu lösen, wodurch dann der Klemmbügel 9 wieder in die Schwenkkonfiguration verstellt ist. Die Ansatzplatte 4 kann nun erneut um die Schwenkachse 6 geschwenkt werden und der Winkel zwischen der Ansatzplatte 4 und dem Hauptkörper 3 kann neu ausgerichtet werden.

Durch das oben beschriebene Verstellen des Klemmbügels 9 zurück in die Klemmkonfiguration kann die Ansatzplatte 4 und damit der Winkel wieder fixiert werden. Im Falle einer irreversiblen Klemmkonfiguration sind am Klemmbügel 9 zwei Sollbruchstellen im Material ausgebildet (beispielsweise Einkerbungen bzw. Schwachstellen), so dass Teile des Klemmbügels 9 abgebrochen werden können, wenn er die Klemmkonfiguration eingenommen hat. Der Klemmbügel 9 kann auch sowohl eine reversible als auch eine irreversible Klemmkonfiguration aufweisen, d.h. der Klemmbügel 9 kann so lange reversibel zwischen der Klemmkonfiguration und der Schwenkkonfiguration verstellt werden, bis der Klemmbügel 9 abgebrochen wird und dann eine irreversible Klemmkonfiguration einnimmt.

### Bezugszeichenliste

- 1: Implantat
- 2: Längsachse
- 3: Hauptkörper
- 4: Ansatzplatte
- 5: Anlagefläche
- 6: Schwenkachse
- 7: Klemmelement
- 8: Klemmfläche
- 9: Klemmbügel
- 10: Fortsatz
- 11: Sicherungsfläche
- 12: Kopplungsglied
- 13: Dorn
- 14: Schwenkeinheit
- 15: Ausbuchtung
- 16: Verbindungsgliedaufnahme
- 17: Ausbuchtungsaufnahme
- 18: Verbindungsgliedöffnung
- 19: Verbindungsglied
- 20: Schwenkmittel
- 21: Klemmelementverbindungsgliedaufnahme
- 22: Klemmelementverbindungsgliedöffnung
- 23: Klemmelementverbindungsglied
- 24: Verstärkung
- 25: Klemmbügelaufnahme
- 26: Klemmbügelfortsatz
- 27: federndes Element
- 28: Mantelfläche
- 29: Ausnehmungen
- 30: Ansatzplattenausnehmung
- 31: Klemmachse
- 32: Klemmbügelschenkel
- 33: Rand
- 34: Zähne

## Patentansprüche

1. Implantat (1) zum Einbringen zwischen Wirbelkörper der Wirbelsäule, mit einem entlang einer Längsachse (2) ausgebildeten Hauptkörper (3), an dessen mindestens einem seiner Enden eine Ansatzplatte (4) mit einer Anlagefläche (5) zur Anlage an einen angrenzenden Wirbelkörper angeordnet ist, die um eine im Wesentlichen senkrecht zur Längsachse (2) angeordnete Schwenkachse (6) schwenkbar am Hauptkörper (3) gelagert und unter einem Winkel gegenüber diesem oder gegenüber der Längsachse (2) fixierbar ist, wobei dem Hauptkörper (3) mindestens ein Klemmelement (7) zugeordnet ist und die Ansatzplatte (4) an einer dem Hauptkörper (3) zugewandten Seite eine Klemmfläche (8) aufweist, wobei das Klemmelement (7) verstellbar ist zwischen einer Schwenkkonfiguration, in der die Ansatzplatte (4) schwenkbar ist, und einer Klemmkonfiguration, in der durch eine Wechselwirkung des Klemmelements (7) mit der Klemmfläche (8) der Winkel und damit die Ansatzplatte (4) fixiert sind, **dadurch gekennzeichnet, dass** das Klemmelement (7) um eine im Wesentlichen senkrecht zur Längsachse (2) angeordneten Achse schwenkbar am Hauptkörper (3) gelagert ist.

2. Implantat (1) nach Anspruch 1, **dadurch gekennzeichnet, dass** das Klemmelement (7) als ein Klemmbügel (9) gebildet ist, der den Hauptkörper (3) umfangsseitig zumindest teilweise umgreift.

3. Implantat (1) nach einem der Ansprüche 1 oder 2, **dadurch gekennzeichnet, dass** am Außenumfang des Hauptkörpers (3) ein Fortsatz (10) ausgebildet oder angeordnet ist, welcher insbesondere eine zumindest teilweise planare Sicherungsfläche (11) umfasst.

4. Implantat (1) nach Anspruch 3, **dadurch gekennzeichnet, dass** der Fortsatz (10) unter einem Neigungswinkel geneigt oder im Wesentlichen parallel zur Längsachse (2) ausgerichtet ist.

5. Implantat (1) nach einem der Ansprüche 1 bis 4, **dadurch gekennzeichnet, dass** dem Klemmelement (7) mindestens ein Kopplungsglied (12) an der der Klemmfläche (8) zugewandten Seite zugeordnet ist zur zusätzlichen Sicherung der Klemmkonfiguration.

6. Implantat (1) nach Anspruch 5, **dadurch gekennzeichnet, dass** das Kopplungsglied (12) als ein einzelner oder als mehrere Dorne (13) gebildet ist.

7. Implantat (1) nach einem der Ansprüche 1 bis 6, **dadurch gekennzeichnet, dass** die Ansatzplatte (4) und der Hauptkörper (3) durch eine Schwenkeinheit (14) miteinander verbunden sind, die eine dem Hauptkörper (3) zugewandte Ausbuchtung (15) mit einer Verbindungsgliedaufnahme (16), die eine am Hauptkörper (3) ausgebildete Ausbuchtungsaufnahme (17) mit einer Verbindungsgliedöffnung (18) und die ein in die Verbindungsgliedaufnahme (16) sowie in die Verbindungsgliedöffnung (18) eingesetztes Verbindungsglied (19) umfasst.

8. Implantat (1) nach Anspruch 7, **dadurch gekennzeichnet, dass** die Ausbuchtung (15) zumindest teilweise konvex gebildet ist, und dass die Ausbuchtungsaufnahme (17) vorzugsweise zumindest teilweise konkav gebildet ist.

9. Implantat (1) nach einem der Ansprüche 1 bis 8, **dadurch gekennzeichnet, dass** das Klemmelement (7) und der Hauptkörper (3) durch ein Schwenkmittel (20) miteinander verbunden sind, das eine am Klemmelement (7) ausgebildete Klemmelementverbindungsgliedaufnahme (21), das eine am Hauptkörper (3) ausgebildete Klemmelementverbindungsgliedöffnung (22) und das ein in die Klemmelementverbindungsgliedaufnahme (21) sowie in die Klemmelementverbindungsgliedöffnung (22) eingesetztes Klemmelementverbindungsglied (23) umfasst.

10. Implantat (1) nach Anspruch 9, **dadurch gekennzeichnet, dass** der Hauptkörper (3) an dem der Schwenkeinheit (14) zugewandten Ende eine Verstärkung (24) aufweist, in der die Ausbuchtungsaufnahme (17) mit der Verbindungsgliedöffnung (18) und die Klemmelementverbindungsgliedöffnung (22) ausgebildet sind.

11. Implantat (1) nach einem der Ansprüche 2 bis 10, **dadurch gekennzeichnet, dass** der Hauptkörper (3) eine Klemmbügelaufnahme (25) aufweist zur Aufnahme eines am Klemmbügel (9) ausgebildeten Klemmbügelfortsatzes (26).

12. Implantat (1) nach einem der Ansprüche 1 bis 11, **dadurch gekennzeichnet, dass** am Hauptkörper (3) ein federndes Element (27) angeordnet ist zur Sicherung des Klemmelements (7) in der Klemmkonfiguration.

13. Implantat (1) nach Anspruch 12, **dadurch gekennzeichnet, dass** das federnde Element (27) in der Schwenkkonfiguration, vorzugsweise radial, vorgespannt ist.

14. Implantat nach Anspruch 12 oder 13, **dadurch gekennzeichnet, dass** das federnde Element (27) in der Schwenkkonfiguration axial auf den Klemmbügelfortsatz (26) wirkt und dass der Klemmbügelfortsatz (26) in der Klemmkonfiguration radial mit der Klemmbügelaufnahme (25) zusammenwirkt.

## Claims

1. An implant (1) for insertion between vertebral bodies of the spinal column, with a main body (3) which is formed along a longitudinal axis (2) and at the at least one end of which an attachment plate (4) with a contact surface (5) for attachment to an adjacent vertebral body is disposed, which plate is mounted on main body (3) pivotably about a pivot axis (6), arranged substantially perpendicular to the longitudinal axis (2), and which can be fixed at an angle relative to it or relative to the longitudinal axis (2), wherein the main body (3) is assigned at least one clamping element (7) and the attachment plate (4) has a clamping surface (8) on a side facing the main body (3), wherein the clamping element (7) is movable between a pivot configuration, in which the attachment plate (4) is pivotable, and a clamping configuration, in which the angle and thereby the attachment plate (4) are fixed by an interaction of the clamping element (7) with the clamping surface (8), **characterized in that** the clamping element (7) is mounted on the main body (3) pivotably about an axis arranged substantially perpendicular to the longitudinal axis (2).

2. The implant (1) according to claim 1, **characterized in that** the clamping element (7) is formed as a clamping bracket (9) which surrounds at least partially the main body (3) circumferentially.

3. The implant (1) according to one of claims 1 or 2, **characterized in that** an extension (10), which comprises in particular an at least partially planar securing surface (11), is formed or disposed on the outer circumference of the main body (3).

4. The implant (1) according to claim 3, **characterized in that** the extension (10) is inclined at a tilt angle or is oriented substantially parallel to the longitudinal axis (2).

5. The implant (1) according to one of claims 1 to 4, **characterized in that** the clamping element (7) is assigned at least one coupling member (12) on the side facing the clamping surface (8) for additional securing of the clamping configuration.

6. The implant (1) according to claim 5, **characterized in that** the coupling member (12) is formed as one barb or more barbs (13).

7. The implant (1) according to one of claims 1 to 6, **characterized in that** the attachment plate (4) and the main body (3) are connected to one another by a pivot unit (14), which comprises a projection (15), facing the main body (3), with a connecting member seat (16), a projection seat (17), formed on the main body (3), with a connecting member opening (18), and a connecting member (19) inserted in the connecting member seat (16) and in the connecting member opening (18).

8. The implant (1) according to claim 7, **characterized in that** the projection (15) is formed at least partially convex and that the projection seat (17) is preferably formed at least partially concave.

9. The implant (1) according to one of claims 1 to 8, **characterized in that** the clamping element (7) and the main body (3) are connected to one another by a pivoting means (20), which comprises a clamping element connecting member seat (21) formed on the clamping element (7), a clamping element connecting member opening (22) formed on the main body (3), and a clamping element connecting member (23) inserted in the clamping element connecting member seat (21) and in the clamping element connecting member opening (22).

10. The implant (1) according to claim 9, **characterized in that** the main body (3) at the end facing the pivot unit (14) has reinforcement (24), in which the projection seat (17) with the connecting member opening (18) and the clamping connecting member opening (22) are formed.

11. The implant (1) according to one of claims 2 to 10, **characterized in that** the main body (3) has a clamping bracket seat (25) for receiving a clamping bracket extension (26) formed on the clamping bracket (9).

12. The implant (1) according to one of claims 1 to 11, **characterized in that** a flexible element (27) is disposed on the main body (3) for securing the clamping element (7) in the clamping configuration.

13. The implant (1) according to claim 12, **characterized in that** the flexible element (27) is pretensioned preferably radially in the pivot configuration.

14. The implant according to claim 12 or 13, **characterized in that** the flexible element (27) in the pivot configuration acts axially on the clamping bracket extension (26) and that the clamping bracket extension (26) in the clamping configuration acts radially together with the clamping bracket seat (25).

## Revendications

1. Implant (1) destiné à être mis en place entre des corps vertébraux du rachis, comprenant un corps principal (3) formé le long d'un axe longitudinal (2), sur au moins une extrémité duquel est disposée une plaque rapportée (4) dotée d'une surface d'appui (5), qui est destinée à l'appui contre un corps vertébral adjacent et qui est montée de façon pivotante sur le corps principal (3), avec possibilité de pivotement autour d'un axe de pivotement (6) disposé de façon sensiblement perpendiculaire à l'axe longitudinal (2), et peut être fixée en formant un angle avec ce corps ou avec l'axe longitudinal (2), sachant qu'au moins un élément de serrage (7) est associé au corps principal (3) et que la plaque rapportée (4) présente une surface de serrage (8) sur une face tournée vers le corps principal (3), sachant que l'élément de serrage (7) peut être réglé entre une configuration de pivotement, dans laquelle la plaque rapportée (4) peut pivoter, et une configuration de serrage, dans laquelle l'angle et donc la plaque rapportée (4) sont fixés sous l'effet d'une interaction entre l'élément de serrage (7) avec la surface de serrage (8), **caractérisé en ce que** l'élément de serrage (7) est monté de façon pivotante sur le corps principal (3), avec possibilité de pivotement autour d'un axe disposé de façon sensiblement perpendiculaire à l'axe longitudinal (2).

2. Implant (1) selon la revendication 1, **caractérisé en ce que** l'élément de serrage (7) est réalisé sous forme d'étrier de serrage (9) qui entoure au moins partiellement le corps principal (3) sur sa circonférence.

3. Implant (1) selon l'une des revendications 1 ou 2, **caractérisé en ce qu'**une saillie (10) est formée ou disposée sur le pourtour du corps principal (3), qui comprend notamment une surface d'arrêt (11) au moins partiellement plane.

4. Implant (1) selon la revendication 3, **caractérisé en ce que** la saillie (10) est inclinée sous un angle d'inclinaison ou est orientée de façon sensiblement parallèle à l'axe longitudinal (2).

5. Implant (1) selon l'une des revendications 1 à 4, **caractérisé en ce qu'**au moins un élément d'accouplement (12) est associé à l'élément de serrage (7), sur le côté tourné vers la surface de serrage (8), en vue du maintien supplémentaire de la configuration de serrage.

6. Implant (1) selon la revendication 5, **caractérisé en ce que** l'élément d'accouplement (12) est réalisé sous la forme d'un ou plusieurs dômes (13).

7. Implant (1) selon l'une des revendications 1 à 6, **caractérisé en ce que** la plaque rapportée (4) et le corps principal (3) sont reliés l'un à l'autre par une unité de pivotement (14) qui comprend un renflement (15) tourné vers le corps principal (3) et pourvu d'un logement recevant un organe d'assemblage (16), qui comprend un logement recevant un renflement (17) formé sur le corps principal (3) et doté d'une ouverture recevant un organe d'assemblage (18), et qui comprend un organe d'assemblage (19) inséré dans le logement recevant un organe d'assemblage (16) ainsi que dans l'ouverture recevant un organe d'assemblage (18).

8. Implant (1) selon la revendication 7, **caractérisé en ce que** le renflement (15) est réalisé au moins en partie sous une forme convexe, et **en ce que** le logement recevant un renflement (17) est réalisé de préférence au moins en partie sous une forme concave.

9. Implant (1) selon l'une des revendications 1 à 8, **caractérisé en ce que** l'élément de serrage (7) et le corps principal (3) sont reliés l'un à l'autre par un moyen de pivotement (20) qui comprend un logement recevant un organe d'assemblage d'élément de serrage (21) réalisé sur l'élément de serrage (7), qui comprend une ouverture recevant un organe d'assemblage d'élément de serrage (22) réalisé sur le corps principal (3), et qui comprend un organe d'assemblage d'élément de serrage (23) inséré dans l'ouverture recevant un organe d'assemblage d'élément de serrage (22).

10. Implant (1) selon la revendication 9, **caractérisé en ce que** le corps principal (3) présente, à l'extrémité tournée vers l'unité de pivotement (14), une zone épaissie (24) dans laquelle sont réalisés le logement recevant le renflement (17), comportant l'ouverture recevant l'organe d'assemblage (18), et l'ouverture recevant l'organe d'assemblage d'élément de serrage (22).

11. Implant (1) selon l'une des revendications 2 à 10, **caractérisé en ce que** le corps principal (3) présente un logement recevant l'étrier de serrage (25), destiné à recevoir une saillie d'étrier de serrage (26) réalisé sur l'étrier de serrage (9).

12. Implant (1) selon l'une des revendications 1 à 11, **caractérisé en ce qu'**il est prévu sur le corps principal (3), un élément faisant ressort (27) destiné à maintenir l'élément de serrage (7) dans la configuration de serrage.

13. Implant (1) selon la revendication 12, **caractérisé en ce que** dans la configuration de pivotement, l'élément faisant ressort (27) est mis sous précontrainte, de préférence radiale.

14. Implant (1) selon la revendication 12 ou 13, **caractérisé en ce que** dans la configuration de pivotement, l'élément faisant ressort (27) agit axialement sur la saillie d'étrier de serrage (26), et **en ce que** dans la configuration de serrage, la saillie d'étrier de serrage (26) coopère radialement avec le logement recevant l'étrier de serrage (25).
